# EUROPEAN PATENT SPECIFICATION

(11) **EP 3 639 879 B1**
(45) Date of publication and mention of the grant of the patent: **11.11.2020**
(21) Application number: 19215522.4
(22) Date of filing: 17.09.2003
(51) Int. Cl.: A61M 16/16, A61M 16/10, A61M 16/00

(54) **APPARATUS FOR DELIVERING HUMIDIFIED GASES**
GERÄT ZUR ABGABE VON BEFEUCHTETEN GASEN
APPAREIL D'ADMINISTRATION DE GAZ HUMIDIFIÉS

(30) Priority: 17.09.2002 NZ 52144602; 20.08.2003 NZ 52773403
(43) Date of publication of application: 22.04.2020
(62) Divisional of application: 03797756.8
(73) Proprietor: Fisher & Paykel Healthcare Limited, East Tamaki Auckland 2013 (NZ)
(72) Inventor: KRAMER, Paul, Friedrich, 1005 Auckland (NZ); MAKINSON, Ian, Douglas, 1003 Auckland (NZ); BIGGS, Philip, James, 1310 Auckland (NZ); DICKINSON, Philip, John, 1005 Auckland (NZ)
(74) Representative: Forresters IP LLP

(56) References cited:
- WO-A2-01/10489
- US-A1- 2001 050 080
- US-B1- 6 397 841

## Description

### BACKGROUND TO THE INVENTION

### i) Field of the Invention

The present invention relates to apparatus for delivering humidified gases. In particular it relates to a humidifier arrangement for an integrated device providing respiratory assistance to patients, for example in consumer CPAP delivery devices.

### ii) Summary of the Prior Art

Humidification systems are known which include a heater base and a disposable humidifier chamber which is fitted onto the heater base and within which a supply of water can be heated by the heater base. Air enters the humidifier chamber through an inlet air port in the roof of the chamber where it is humidified by the evaporation of water from the water supply before leaving the chamber through an exit port in the roof of the humidifier chamber.

Humidifier chambers of this type are also now used in compact and portable ventilation machines, for example machines intended for the home treatment of obstructive sleep apnoea (CPAP machines). Where the humidifier base is adapted for use with slide-on humidifier chambers, and the connection of the chamber to the machine is accomplished with a single sliding movement, the inlet air port is provided horizontally through the side of the chamber. Air enters the humidifier chamber through the inlet air port and the humidified air leaves the humidifier chamber into a breathing conduit through an exit port in the top of the humidifier chamber.

A disadvantage of these configurations is the need to disconnect the patient breathing conduit from the top of the humidifying chamber in a separate operation before removal of the chamber for the purpose of refilling. A further disadvantage of these configurations is that separate electrical wiring connections are required to make use of a heated respiratory conduit.

The present invention is described with particular reference to a CPAP delivery product. However it will be appreciated that the invention is applicable to any compact integrated humidified gases delivery product incorporating a pressurised gases supply and a humidification module. For example, physically similar devices may be used for patient ventilation, humidified oxygen delivery, and humidified insufflation.

US 2001/050080 A1 discloses a conventional humidifier and humidity sensor for use with a breathing assistance apparatus.

### SUMMARY OF THE INVENTION

It is an object of the present invention to provide an apparatus for delivering humidified gases which at least goes some way towards overcoming the above disadvantages or which will at least provide the public with a useful choice.

According to one aspect of the present invention, there is provided an apparatus as defined in claim 1 hereinafter.

To those skilled in the art to which the invention relates, many changes in construction and widely differing embodiments and applications of the invention will suggest themselves without departing from the scope of the invention as defined in the appended claims. The disclosures and the descriptions herein are purely illustrative and are not intended to be in any sense limiting.

### BRIEF DESCRIPTION OF THE DRAWINGS

Embodiments of the present invention will now be described with reference to the drawings.
Figure 1 is a perspective view of a water chamber and CPAP machine according to an embodiment of the present invention showing the water chamber 2 separated from the CPAP machine 1.
Figure 2 is a perspective view of the water chamber and CPAP machine of Figure 1, showing the water chamber 2 engaged with the CPAP machine 1.
Figure 3 is a perspective view of a CPAP machine and water chamber according to an alternative embodiment of the present invention.
Figure 4 is a perspective view of a water chamber of the present invention showing hidden detail of the inlet and outlet extension tubes.
Figure 5 is a sectioned side view of the water chamber of Figure 4 sectioned through a mid-line of the outlet extension tube with the intended water level shown hatched.
Figure 6 is a sectioned side view of the water chamber of Figure 4, sectioned through a mid-line of the chamber with the water level of the chamber when tilted shown hatched.
Figure 7 is a perspective view of an inlet/outlet extension tube according to an embodiment of the present invention showing snap-fit protrusions and locating/locking means.
Figure 8 is a front view of a water chamber of the present invention showing the flanges and notches which co-operate with the extension tubes detailed in Figure 7.
Figure 9 is a perspective view of an outlet extension tube according to an embodiment of the present invention showing an air bleed slot.
Figure 10 is a perspective view of a water chamber according to a further embodiment showing hidden detail of the inlet and outlet extension tubes.

### DETAILED DESCRIPTION

Embodiments of the present invention will now be described in more detail.

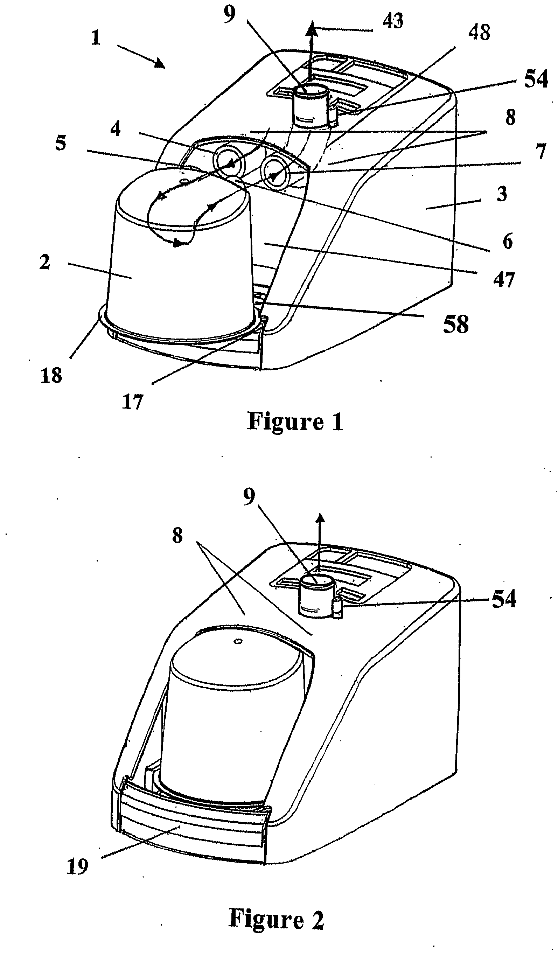

Referring to Figures 1 and 2, a preferred embodiment of the invention, in a CPAP machine has a housing containing a blower and a heater base, and a corresponding water chamber. A water chamber having a gases inlet port 5 and gases outlet port 6 is shown with a portable CPAP machine. The CPAP machine is adapted to receive slide-on humidifier chambers. The CPAP machine connects to the gases inlet/outlet ports of the water chamber through a connection manifold. Connection of the gases inlet and gases outlet ports are made to the connection manifold 8 of the CPAP machine in a single slide-on motion. The connection manifold 8 also provides an auxiliary outlet connection port 9 suitable for receiving a flexible respiratory conduit to deliver humidified air to a patient.

The CPAP machine includes a heater base 58 in a chamber receiving bay 47 to heat the water chamber. A securing arrangement is provided for locating and engaging the water chamber to the CPAP machine. The securing arrangement has a securing latch 19 and a slot 17 around the periphery of the chamber receiving bay 47. The slot cooperates with a flange 18 around the base of the water chamber to secure the chamber when in use. The securing latch 19 operates to prevent removal of the chamber once it has been engaged. The securing means and connection manifold are arranged with a parallel axis of operation, such that connection of the chamber inlet and outlet ports 5 & 6, to the connection manifold 8 is achieved together with the securing of the chamber into the CPAP machine in the same single slide-on motion. The insertion direction of the connectors for ports 5, 6 is the same as at least the terminal part of the slide-on motion.

The latch 19, having a locking position and a release position, is biased toward the locking position which prevents removal of the chamber from the CPAP machine. The front face of the latch may be shaped such that during the single slide-on motion employed to fit the water chamber to the CPAP machine, the flange 18 urges the securing latch 19 into the release position and allows the water chamber to be properly fitted. Once the base of the water chamber is properly seated on the heater base and the inlet 5 and outlet 6 are properly engaged with the connection manifold 8, the flange 18 and base of the chamber no longer contact the securing latch 19. This allows the securing latch biasing means to urge the latch into the locking position and prevent the water chamber from being removed as shown in Figure 2.

Preferably the connection manifold 8 includes a passage which receives pressurised airflow from the blower and directs it into the water chamber 2, and a passage which directs airflow received via the water chamber outlet port 6, to the CPAP patient outlet port 9. The connection passage connecting the manifold inlet port 7, to the manifold patient outlet port 9 is shown in hidden detail 48 in Figure 1. The connection manifold 8 of the present invention is preferably embodied in a removable component to aid cleaning and/or sterilisation of the gases passageways. In one preferred embodiment the above connection passages are internal to the connection manifold 8 as illustrated in Figures 1 and 2.

In use, air from the CPAP machine blower exits through outlet port 4, and enters the chamber 2 through inlet port 5. A chamber heating means 58 vaporises liquid water in the chamber, and air entering the chamber is humidified by the evaporation of water from the water source in the bottom of the chamber before leaving the chamber through the patient outlet port 6. Humidified air from the outlet port 6 is received into the connection manifold of the CPAP machine 8 via the inlet port 7. The connection manifold 8 directs air to the outlet port 9 which is adapted to connect with a flexible conduit connector for delivery to a patient. An advantage obtained from the breathing conduit connection 9 being located on the body of the CPAP machine and not connected to the top of the water chamber directly, is that complete connection or disconnection of the water chamber from the CPAP system (including the breathing conduit) can be achieved with a single slide-on or slide-off motion respectively. This feature simplifies removal of the water chamber for refilling compared with prior art devices.

A further advantage is obtained when additional electrical or pneumatic connections are required for example for heated delivery conduits. The use of heated conduits usually requires electrical wiring connectors between the conduit and humidified air source while an additional pneumatic connection may be used for pressure feedback or measurement. In the present invention the connector may include any additional electrical and/or pneumatic 54 connection for the conduit. The connector is integral to the connection manifold of the CPAP machine 8 and therefore allows the disposable water chamber to remain simple for example lacking electrical transfer connections.

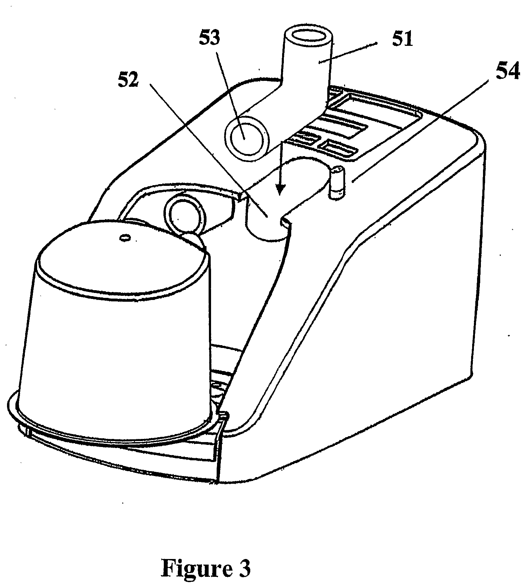

A number of alternative variations of the present invention are envisaged and will now be described. For example, a further embodiment of the present invention is envisaged to deliver humidified gases from the water chamber to a patient via a flexible breathing conduit wherein the humidified gases portion of the manifold is separately removable from the housing. This alternative embodiment is shown in Figure 3. An elbow tube 51 having an inlet end and an outlet end is provided to receive humidified gases from the water chamber and direct humidified gases into a flexible breathing conduit for delivery to a patient. In this alternative embodiment the CPAP machine housing is provided with a recess 52 for receiving and securing the elbow tube. The recess 52 may include a neck or constriction above the elbow 51, (when elbow 51 is in place) to hold the elbow in place under normal usage, but also allow the elbow to be removed when required. It will be appreciated that other methods of removably securing elbow 51, will readily present themselves to those skilled in the art. For example via various protrusions and interacting slots on one or other of elbow 51, or around recess 8, or both. When secured in position, an inlet 53 of the elbow tube 51 is positioned to make a fluid connection to the outlet 6 of the water chamber in the same slide on motion. In this alternative embodiment the outlet elbow may be part of the termination of the breathing tube instead of an internal part of the connection manifold as previously described. An advantage of this alternative embodiment is that the parts in contact with potential condensation are removable for cleaning and/or sterilisation. This embodiment also retains the advantage of an engagable/disengagable water chamber in a single slide on/off motion. This embodiment may also allow additional electrical or pneumatic connections 54 between the CPAP machine and a conduit connector to be made directly to the housing enabling this alternative to retain the advantages of the previously described embodiments.

An alternative embodiment of the present invention is envisaged wherein a water chamber and heater base are partially or fully enclosed in a housing. The housing includes a connection manifold consisting of at least one gases inlet and at least one gases outlet connection port being adjacent and aligned, which in use transport gases to and/or from the water chamber. A second housing is provided with complementary inlet and outlet connections for registration with the connection manifold. The second housing is adapted to engage with the first housing making all the necessary gases and electrical or pneumatic connections in the same slide-on motion and preferably includes a securing arrangement to lock the two housings together. The second housing may include an integral air blower, and a patient conduit outlet port in the case of a CPAP embodiment. The first conduit port in use receiving air from a source and the second conduit port delivering humidified air to a patient. The above described embodiment has the advantage that all necessary flexible conduit connections are made on the second housing (incorporating the gases supply). This enables the water chamber and/or enclosing housing to be removed/engaged in the same slide-off/on motion making engagement/disengagement and refilling of the chamber simpler.

In the preferred embodiments of the present invention, tubular protrusions (4, 7) are provided for making a connection between the humidifier apparatus and a water chamber in order to deliver gases to the chamber and receive humidified gases from the chamber. Preferably the tubular protrusions also include a resilient boot in order to provide an improved seal between the water chamber and the protrusions.

A further embodiment of the present invention is envisaged wherein the connections between the apparatus manifold and the water chamber are not provided side by side, but rather are provided one within the other, for example the inlet and outlet may be coaxial. Such a configuration would enjoy the same advantages as the configurations described in more detail in the preferred embodiments of the present invention. It is also envisaged that such connections may also include similarly configured tubes for providing pressure measurements or pressure feedback as well as electrical connections.

While the above preferred embodiments describe male/female type complimentary connectors wherein the water chamber has two female connectors for mating with corresponding male connectors of the apparatus manifold, many variations will present themselves to those skilled in the art. For example the water chamber may be provided with two male connectors while the apparatus manifold is provided with corresponding female connectors, or the water chamber may be provided with one male and one female connector for connecting to the corresponding male and female connectors of the apparatus manifold. Further it is envisaged that connectors of an androgynous nature may be provided for making connection between the water chamber and the apparatus manifold wherein each connector may include both male type protruding portions and female type recess portions. Such connections may be particularly advantageous when the inlet and outlet is provided one within the other.

With reference to the above embodiments of the present invention, some common features of a water chamber suitable for use with the embodiments described above will now be described in more detail.

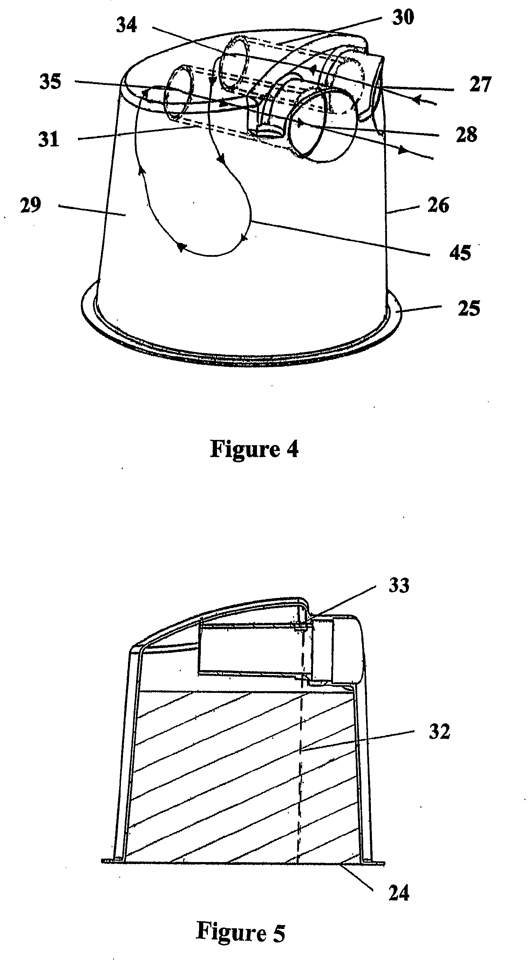

The chamber as shown in Figure 4 and Figure 5 is constructed from an open bottomed plastic container enclosed by a heat conductive base 24, and includes a horizontally aligned gases inlet 27 and a parallel gases outlet 28. It is envisaged that other configurations of the present invention are possible where the slide-on direction employed to fit the water chamber is not horizontal but at an angle from the horizontal or vertical. In such cases, the gases inlet 27 and outlet 28, are preferably parallel and aligned with the direction of the intended slide-on motion to allow mating of the chamber inlet/outlet ports and the connection manifold.

The water chamber of the present invention preferably includes at least one flow tube, being an inlet extension tube 30, and/or an outlet extension tube 31, extending inwardly into the chamber interior from the periphery of the chamber wall and preferably having a generally tapering body. The inlet extension tube 30 and the outlet extension tube 31 are preferably moulded from the same clear thermoplastic material as the chamber shell 26. The inclusion of inlet/outlet extension tubes has been found to significantly reduce noise produced by the airflow around the chamber. However at high flow rates, it is possible for water droplets or splashes to become entrained in the air flow and be carried out the chamber outlet 28. This is especially possible when the water chamber contains a large amount of liquid and the water surface is closer to the chamber outlet. This situation has the potential to become more problematic if the outlet port of the CPAP machine is disconnected from the patient delivery conduit, lowering the circuit resistance and resulting in significantly higher flow rates. Further, without the delivery conduit connected, any liquid entrained in the gases flow may be ejected directly from the chamber. This difficulty may be alleviated somewhat in chambers incorporating various extension tube configurations.

Preferably at least one extension tube has an air bleed aperture 33 to aid filling of the chamber with the chamber tipped up. The air bleed is preferably located in the top surface of the extension tube and preferably toward the end of the extension tube which is connected to the chamber wall. Referring to Figure 5, preferably the air bleed aperture 33 is positioned such that when the tank is tipped up for filling, the air bleed valve height corresponds with the preferred fill height 32 for the water chamber. This feature aids in preventing overfilling of the water chamber.

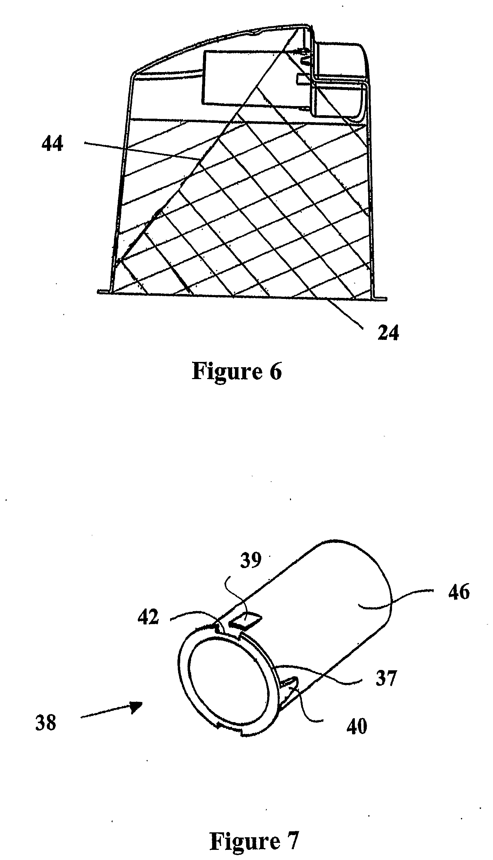

Additionally, with reference to Figure 6, the extension tubes 30 and 31 may act as a weir against water flow back through the gases inlet and gases outlet, upon tilting of the chamber as shown by water level line 44. This reduces water back-flow through the inlet port 27 occurring upon tilting of the chamber. If present, preferably the air bleed aperture 33 is present only on the outlet extension tube 31 and not present in the inlet extension tube 30. Alternatively the air bleed aperture may be included on both.

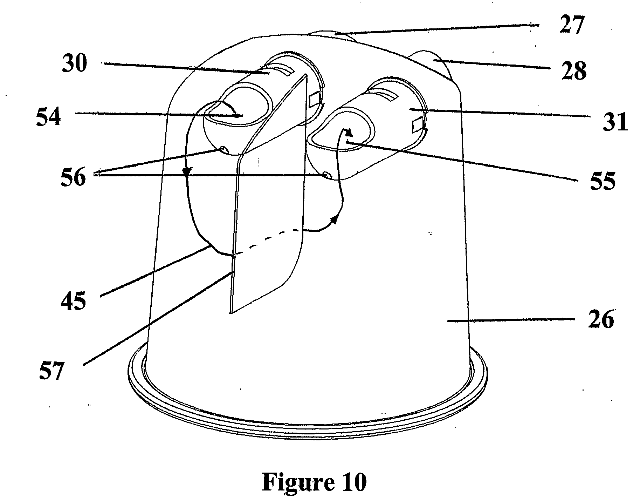

With reference to Figure 10, the present invention may further include a downwardly extending central baffle or rib 57 located between the inlet and outlet extension tubes to ensure against gases short circuiting the chamber by flowing directly from the exit of the inlet extension tube, to the entry of the outlet extension tube. With the baffle present, the gases are forced to follow a more tortuous path ensuring adequate humidification during their journey through the chamber but without increasing the pressure losses in the chamber to an unacceptable level. The baffle preferably extends downwards from the roof of the chamber, and inwards from the portion of the chamber wall opposite the inlet/outlet port. Preferably the size of the baffle is such that it not only ensures that the gases flow follows a torturous path through the chamber, but also provides an additional barrier to splashes entering the inlet 55 of the outlet extension tube 31. As the risk of splashes entering the extension tubes is highest when the water level is highest, the baffle may extend downwards such that it terminates below the water line when the chamber is full.

With reference to Figure 4, in use air is received into the chamber via inlet port 27 and travels down the inlet extension tube 30. On exiting the inlet extension tube 30 air enters the chamber where it is humidified by the evaporation of water from the water supply. Humidified air flows from the chamber through the outlet extension tube 31 and exits through outlet port 28 as illustrated by arrow 45. With reference to Figure 10, an alternative configuration of the extension tubes wherein the distal end of the extension tube furthest from the gases inlet 27 and gases outlet 28 respectively are directed away from the axis of the extension tube. The extension tubes are shaped to minimise the internal pressure losses of the gases flowing through the chamber in order to improve the efficiency of the chamber. In use, air is received into the chamber via inlet port 27 and travels down the inlet extension tube 30. On exiting the upwardly facing outlet 54 of the inlet extension tube 30, the gases flow is directed away from the surface of the water in the chamber, minimising the potential for splashing or water entrainment to occur. As the gases flow enters the chamber it is deflected off the roof of the chamber and is humidified by the evaporation of water from the water supply. Humidified air flows from the chamber through the upwardly facing inlet 55 of the outlet extension tube 31 and exits through outlet port 28. The upwardly oriented inlet 55 of the outlet extension tube 31 eliminates the direct path that splashes might have from the surface of the water into the outlet port 28. A drain hole 56 is provided in the bottom of the extension tubes to enable water to drain back into the chamber after filling, or built up condensation or splashes to drain during use. Preferably the shape and orientation of the extension tube and the position of the drain hole are such that the drain hole is at a low point and fluid flows toward the drain hole and back into the chamber.

Alternatively, it is envisaged that the direction in which the outlet of the inlet extension tube and/or the inlet of the outlet extension tube, faces could be varied in order to achieve differing results. For example, the openings at the distal ends of the extension tubes may be rotated about the axis of the extension tube, to face in any direction. Further, the direction in which the openings of the inlet and outlet flow tubes face may not be the same. Such arrangements (for example facing mutually away from each other) maybe particularly suited for reducing the potential for splashes, and reducing the potential for splashes to enter the opening of the extension tubes when the baffle is present. Although the preceding description gives details of preferred embodiments having parallel and adjacent circular inlet/outlet ports, it is envisaged that other configurations are possible. For example the inlet/outlet ports of the chamber and connection manifold may have a non-circular cross section and not be symmetrical. Further it is possible that the position of the inlet port with respect to the outlet may take one of many alternative configurations. For example the ports and there corresponding connections may also be co-axial or off-set, one inside the other.

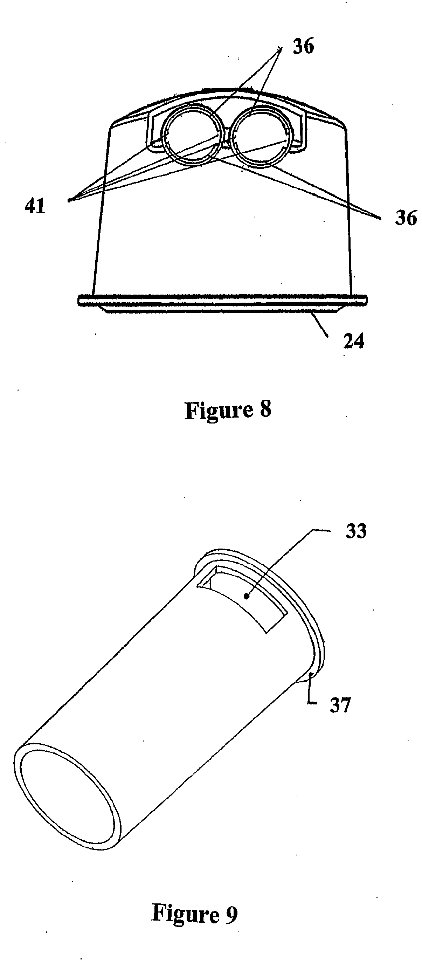

Referring to Figures 7-9, for ease of assembly the inlet and outlet extension tubes are preferably provided as a snap fit to their respective water chamber inlet or outlet, so that they can be pushed into the chamber through the inlet or outlet and, upon application of sufficient force, snap into a substantially watertight and secure condition.

To this end the inlet 27 and outlet 28 ports of the water chamber may be provided with an inwardly perpendicularly extending annular flange 36 at the inner end thereof and the inlet/outlet extension tubes 38 may include similar perpendicularly outwardly extending flanges 37 from one end of the generally tapering tubular body 46. The flanges act together as sealing flanges in the fitted and assembled condition. To retain the extension tubes in the assembled condition, against both translational and rotational movement several securing mechanisms may be provided. In each case the securing mechanisms may be provided on either of the inlet/outlet (of the chamber) or the inlet/outlet extension tube. However it is preferred that they be on the extension tubes, as both components are intended for injection moulding and injection moulding of certain protrusions on the inner surface of the chamber inlet/outlet would be considerably more difficult than on the outer surface of the extension tubes. To secure the tubes against translational movement, and in a sealing condition between the sealing flanges, a plurality of retaining clip protrusions 39 may be provided spaced around the circumference of the tubular body of the extension tubes which co-operate with the inlet/outlet flange 36. Particularly for ease of manufacture, and ensuring a simple two part injection mould, a notch 42 is allowed in the flange 37 of the extension tubes 38 adjacent the protrusion 39.

To retain the extension tubes against rotational movement when snap fitted into location, one or more locating protrusions 40 may be provided circumferentially distributed on the outer surface of the tubular body adjacent and contiguous with the outwardly and perpendicularly extending flange 37. The locating protrusions 40 are preferably generally tapered in both the circumferential and axial direction. Complementary notches 41 are provided in the inwardly extending flanges 36 of the chamber inlet and outlet. In fitting the extension tubes 38 the protrusions 40 are aligned with the notches 41, and upon full insertion of the tubes, the protrusions 40 enter into a tight frictional fit with the notches 41 ensuring substantial if not complete sealing. It will be appreciated that the mechanism employed to ensure proper location and sealing of the extension tubes into the water chamber may take many forms. Many alternatives will suggest themselves to persons skilled in the art such as glued joints, various forms of plastic welding and various configurations of clipping means and protrusions. The above description is of one particular preferred embodiment and is not meant to be in any way limiting.

It will be readily appreciated that the construction of the water chamber as described is simple to manufacture and each of the plastic components is itself capable of simple injection moulding. Consequently a water chamber according to the present invention is, while providing significant advantages, not significantly more expensive than existing chambers.

## Claims

1. An apparatus (1) for use in humidified gases delivery treatment comprising:
a housing,
a pressurised gases supply within said housing,
a pressurised gases outlet in said housing in fluid connection with said pressurised gases supply and adapted to make fluid connection with an inlet of a water chamber in order to provide gases flow to a said water chamber (2),
a humidified gases return in said housing, adapted to make fluid connection with an outlet (6) of a said water chamber (2) in order to receive humidified gases from said water chamber (2),
a patient outlet (9) in said housing, in fluid connection with said humidified gases return in order to receive humidified gases from said humidified gases return and provide humidified gases to said patient outlet (9), said patient outlet (9) being in fluid connection with or adapted to make fluid connection with a flexible breathing conduit for delivery of humidified gases to a patient;
wherein the humidified gases return comprises an elbow tube (51) having an inlet end (53) and an outlet end, provided to receive humidified gases from the water chamber (2) and direct humidified gases into the flexible breathing conduit;
wherein the elbow tube (51) is removably secured on the housing.

2. An apparatus (1) for use in humidified gases delivery treatment as claimed in claim 1 wherein the housing is provided with a recess (52) for receiving and securing the elbow tube (51).

3. An apparatus (1) for use in humidified gases delivery treatment as claimed in claim 2 wherein the recess (52) includes a neck or constriction above the elbow tube (51), when the elbow tube (51) is in place, to hold the elbow tube (51) in place under normal usage, but also allow the elbow tube (51) to be removed when required.

4. An apparatus (1) for use in humidified gases delivery treatment as claimed in claim 2 wherein the elbow tube (51) is removably secured using protrusions and interacting slots on one or other of elbow tube (51), or around recess (52), or both.

5. The apparatus (1) for use in humidified gases delivery treatment as claimed in any one of the preceding claims, comprising a connection manifold (8), wherein the apparatus connects to the inlet (5) and outlet (6) of the water chamber (2) through the connection manifold (8).

6. The apparatus (1) for use in humidified gases delivery treatment as claimed in claim 5 wherein the connection manifold (8) includes a passage which is adapted to receive a pressurised airflow from the pressurised gases supply and to direct the airflow into the water chamber (2), and a passage which is adapted to direct airflow received via the water chamber (2) outlet (6), to the humidified gases return.

7. The apparatus (1) for use in humidified gases delivery treatment as claimed in claim 6 wherein the passages are internal to the connection manifold (8).

8. The apparatus (1) for use in humidified gases delivery treatment of claim 5 wherein the humidified gases return of the connection manifold (8) is separately removable from the housing.

9. The apparatus (1) for use in humidified gases delivery treatment of claim 5 wherein the connection manifold (8) comprises an integral connector, the connector comprising an additional electrical and/or pneumatic connection for the conduit.

10. An apparatus (1) for use in humidified gases delivery treatment as claimed in any one of the preceding claims wherein said patient outlet includes a connector for receiving a breathing hose and at least one auxiliary electrical connection plug or socket or pneumatic connection plug or port, for a simultaneous connection when connecting a breathing circuit having complementary electrical or pneumatic connectors.

11. An apparatus (1) for use in humidified gases delivery treatment as claimed in any one of the preceding claims, further comprising the flexible breathing conduit, wherein the elbow tube (51) comprises part of a termination of the flexible breathing conduit.

12. The apparatus (1) for use in humidified gases delivery treatment as claimed in claim 1 wherein the pressurised gases outlet (4) and the humidified gases return (7) comprise respective tubular protrusions for making the fluid connection between the apparatus and the water chamber (2), wherein the tubular protrusions (4, 7) further comprise a resilient boot in order to provide an improved seal between the water chamber (2) and the protrusions (4, 7).

13. An apparatus (1) for use in humidified gases delivery treatment as claimed in any one of the preceding claims wherein the pressurised gases supply comprises a blower.

14. An apparatus (1) for use in humidified gases delivery treatment as claimed in any one of the preceding claims wherein said water chamber (2) has a base, and said water chamber (2) is engageable with said housing via a single motion.

15. An apparatus for use in humidified gases delivery treatment as claimed in claim 14 wherein said single motion of engagement urges the base of said water chamber (2) adjacent and in contact with said chamber heating means and makes a first fluid connection between said pressurised gases outlet and said water chamber inlet, and makes a second fluid connection between said humidified gases return and said water chamber outlet (6), with said first and second fluid connections being made in the direction of said single motion.

16. The apparatus (1) for use in humidified gases delivery treatment of any one of the preceding claims, comprising:
a water chamber (2); and
a breathing circuit having complementary electrical connectors.

17. The apparatus (1) of any one of the preceding claims configured for humidified oxygen delivery.

18. The apparatus (1) of any one of the preceding claims configured for CPAP therapy.

## Patentansprüche

1. Gerät (1) zur Verwendung bei befeuchtete Gase abgebender Behandlung,
umfassend:
Ein Gehäuse,
eine Druckgasversorgung innerhalb des Gehäuses,
einen Druckgasauslass im Gehäuse in Fluidverbindung mit der Druckgasversorgung und dafür ausgelegt, Fluidverbindung mit einem Einlass einer Writer-Kammer herzustellen, um Gasströmung zu einer besagten Wasserkammer (2) bereitzustellen,
einen Rücklauf für befeuchtete Gase im Gehäuse, dafür ausgelegt, Fluidverbindung mit einem Auslass (6) von einer besagten Wasserkammer (2) herzustellen, um befeuchtete Gase aus der Wasserkammer (2) zu empfangen,
einen Patientenauslass (9) im Gehäuse, in Fluidverbindung mit dem Rücklauf für befeuchtete Gase, um befeuchtete Gase aus dem Rücklauf für befeuchtete Gase zu empfangen und dem Patientenauslass (9) befeuchtete Gase bereitzustellen, wobei der Patientenauslass (9) in Fluidverbindung mit oder für die Herstellung einer Fluidverbindung mit einer Atemleitung zur Abgabe von befeuchteten Gasen an einen Patienten ausgelegt ist;
wobei der Rücklauf für befeuchtete Gase ein Bogenrohr (51) umfasst, das ein Einlassende (53) und ein Auslassende aufweist, das bereitgestellt ist, befeuchtete Gase aus der Wasserkammer (2) zu empfangen und befeuchtete Gase in die flexible Atemleitung zu leiten;
wobei das Bogenrohr (51) entfernbar am Gehäuse befestigt ist.

2. Gerät (1) zur Verwendung bei befeuchtete Gase abgebender Behandlung wie in Anspruch 1 beansprucht, wobei das Gehäuse mit einer Vertiefung (52) zur Aufnahme und Befestigung des Bogenrohrs (51) versehen ist.

3. Gerät (1) zur Verwendung bei befeuchtete Gase abgebender Behandlung wie in Anspruch 2 beansprucht, wobei die Vertiefung (52) einen Hals oder eine Verengung oberhalb des Bogenrohrs (51) einschließt, wenn das Bogenrohr (51) am Ort ist, um das Bogenrohr (51) bei normaler Verwendung in Position zu halten, aber außerdem das Entfernen des Bogenrohrs (51), wenn erforderlich, zu ermöglichen.

4. Gerät (1) zur Verwendung bei befeuchtete Gase abgebender Behandlung wie in Anspruch 2 beansprucht, wobei das Bogenrohr (51), unter Verwendung von Vorsprüngen und aufeinander einwirkenden Schlitzen, an einem oder anderem des Bogenrohrs (51) oder um die Vertiefung (52) oder beiden entfernbar befestigt ist.

5. Gerät (1) zur Verwendung bei befeuchtete Gase abgebender Behandlung wie in einem der vorhergehenden Ansprüche beansprucht, das einen Anschlussverteiler (8) umfasst, wobei sich das Gerät mit dem Einlass (5) und dem Auslass (6) der Wasserkammer (2) durch den Anschlussverteiler (8) verbindet.

6. Gerät (1) zur Verwendung bei der befeuchtete Gase abgebender Behandlung wie in Anspruch 5 beansprucht, wobei der Anschlussverteiler (8) einen Durchgang, der ausgelegt ist, einen unter Druck stehenden Luftstrom von der Druckgasversorgung zu empfangen und den Luftstrom in die Wasserkammer (2) zu leiten, und einen Durchgang einschließt, der ausgelegt ist, den über den Auslass (6) der Wasserkammer (2) empfangenen Luftstrom zum Rücklauf für befeuchtete Gase zu leiten.

7. Gerät (1) zur Verwendung bei befeuchtete Gase abgebender Behandlung wie in Anspruch 6 beansprucht, wobei sich die Durchgänge im Innern des Verbindungsverteilers (8) befinden.

8. Gerät (1) zur Verwendung bei befeuchtete Gase abgebender Behandlung wie in Anspruch 5, wobei der Rücklauf für befeuchtete Gase des Verbindungsverteilers (8) separate vom Gehäuse entfernbar ist.

9. Gerät (1) zur Verwendung bei befeuchtete Gase abgebender Behandlung von Anspruch 8, wobei der Anschlussverteiler (8) einen integralen Verbinder umfasst, wobei der Verbinder einen zusätzlichen elektrischen und/oder pneumatischen Anschluss für die Leitung umfasst.

10. Gerät (1) zur Verwendung bei befeuchtete Gase abgebender Behandlung wie in einem der vorhergehenden Ansprüche beansprucht, wobei der Patientenauslass einen Verbinder zur Aufnahme eines Beatmungsschlauches und zumindest eines zusätzlichen elektrischen Verbindungssteckers oder einer Steckdose oder eines pneumatischen Verbindungssteckers oder einem Port, für eine simultane Verbindung einschließt, wenn ein Atemkreislauf angeschlossen wird, der komplementäre elektrische oder pneumatische Verbinder aufweist.

11. Gerät (1) zur Verwendung bei befeuchtete Gase abgebender Behandlung wie in einem der vorhergehenden Ansprüche beansprucht, das ferner die flexible Atemleitung umfasst, wobei das Bogenrohr (51) einen Teil eines Anschlusses der flexiblen Atemleitung umfasst.

12. Gerät (1) zur Verwendung bei befeuchtete Gase abgebender Behandlung wie in Anspruch 1 beansprucht, wobei der Druckgasauslass (4) und der Rücklauf (7) für befeuchtete Gase jeweilige röhrenförmige Vorsprünge zur Herstellung der Fluidverbindung zwischen dem Gerät und der Wasserkammer (2) umfassen, wobei die rohrförmigen Vorsprünge (4, 7) ferner eine elastische Muffe umfasst, um eine verbesserte Dichtung zwischen der Wasserkammer (2) und den Vorsprüngen (4, 7) bereitzustellen.

13. Gerät (1) zur Verwendung bei befeuchtete Gase abgebender Behandlung wie in einem der vorhergehenden Ansprüche beansprucht, wobei die Druckgasversorgung ein Gebläse umfasst.

14. Gerät (1) zur Verwendung bei befeuchtete Gase abgebender Behandlung wie in einem der vorhergehenden Ansprüche beansprucht, wobei die Wasserkammer (2) eine Basis aufweist, und die Wasserkammer (2) durch eine einzelne Bewegung mit dem Gehäuse in Eingriff bringbar ist.

15. Gerät (1) zur Verwendung bei befeuchtete Gase abgebender Behandlung wie in Anspruch 14 beansprucht, wobei die einzelne Bewegung des Eingreifens die Basis der Wasserkammer (2) benachbart und in Kontakt mit dem Kammerheizmittel drängt und eine erste Fluidverbindung zwischen dem Druckgasauslass und dem Wasserkammereinlass herstellt, und eine zweite Fluidverbindung zwischen dem Rücklauf für befeuchtete Gase und dem Wasserkammerauslass (6) herstellt, wobei die ersten und zweiten Fluidverbindungen in Richtung der einzelnen Bewegung gemacht werden.

16. Gerät (1) zur Verwendung bei befeuchtete Gase abgebender Behandlung nach einem der vorhergehenden Ansprüche, umfassend:
Eine Wasserkammer (2); und
einen Atemkreislauf, der komplementäre elektrische Anschlüsse aufweist.

17. Gerät (1) nach einem der vorhergehenden Ansprüche, das zur Lieferung von befeuchtetem Sauerstoff ausgelegt ist.

18. Gerät (1) nach einem der vorhergehenden Ansprüche, das für CPAP-Therapie ausgelegt ist.

## Revendications

1. Un appareil (1) à utiliser dans un traitement de distribution de gaz humidifiés, comprenant :
un boîtier,
une alimentation en gaz sous pression à l'intérieur dudit boîtier,
un retour de gaz humidifiés dans ledit boîtier en connexion fluidique avec ladite alimentation en gaz sous pression et adapté pour former une connexion fluidique avec une entrée d'une chambre d'eau afin de fournir un flux de gaz à ladite chambre (2),
un retour de gaz humidifiés dans ledit boîtier, adapté pour former une connexion fluidique avec une sortie (6) de ladite chambre d'eau (2) afin de recevoir des gaz humidifiés venant de ladite chambre d'eau (2),
une sortie côté patient (9) dans ledit boîtier, en connexion fluidique avec ledit retour de gaz humidifiés afin de recevoir des gaz humidifiés dudit retour de gaz humidifiés et de fournir des gaz humidifiés à ladite sortie côté patient (9), ladite sortie côté patient (9) étant en connexion fluidique ou adaptée pour former une connexion fluidique avec un conduit respiratoire pour la distribution de gaz humidifiés à un patient ;
dans lequel le retour de gaz humidifiés comprend un tube coudé (51) ayant une extrémité d'entrée (53) et une extrémité de sortie, fourni afin de recevoir des gaz humidifiés de la chambre d'eau (2) et diriger les gaz humidifiés dans le conduit respiratoire flexible ;
dans lequel le tube coudé (51) est fixé de manière amovible sur le boîtier.

2. Un appareil (1) à utiliser dans un traitement de distribution de gaz humidifiés selon la revendication 1, dans lequel le boîtier est muni d'un renfoncement (52) pour recevoir et fixer le tube coudé (51).

3. Un appareil (1) à utiliser dans un traitement de distribution de gaz humidifiés selon la revendication 2, dans lequel le renfoncement (52) inclut un col ou une constriction au-dessus du tube coudé (51), quand le tube coudé (51) est en place, pour maintenir le tube coudé (51) en place lors d'un usage normal, mais aussi pour permettre au tube coudé (51) d'être retiré lorsque nécessaire.

4. Un appareil (1) à utiliser dans un traitement de distribution de gaz humidifiés selon la revendication 2, dans lequel le tube coudé (51) est fixé de manière amovible à l'aide de parties en saillie et de fentes coopérant soit sur le tube coudé (51), soit autour d'un renfoncement (52), soit les deux.

5. L'appareil (1) à utiliser dans un traitement de distribution de gaz humidifiés selon l'une quelconque des revendications précédentes, comprenant un collecteur de connexion (8), dans lequel l'appareil se connecte à l'entrée (5) et à la sortie (6) de la chambre d'eau (2) par le collecteur de connexion (8).

6. L'appareil (1) à utiliser dans un traitement de distribution de gaz humidifiés selon la revendication 5, dans lequel le collecteur de connexion (8) inclut un passage qui est adapté pour recevoir un flux d'air sous pression de l'alimentation en gaz sous pression et pour diriger le flux d'air dans la chambre d'eau (2), et un passage qui est adapté pour diriger le flux d'air reçu par la sortie (6) de la chambre d'eau (2), vers le retour de gaz humidifiés.

7. L'appareil (1) à utiliser dans un traitement de distribution de gaz humidifiés selon la revendication 6, dans lequel les passages sont à l'intérieur du collecteur de connexion (8).

8. L'appareil (1) à utiliser dans un traitement de distribution de gaz humidifiés selon la revendication 5, dans lequel le retour de gaz humidifiés du collecteur de connexion (8) peut être retiré séparément du boîtier.

9. L'appareil (1) à utiliser dans un traitement de distribution de gaz humidifiés selon la revendication 5, dans lequel le collecteur de connexion (8) comprend un connecteur intégré, le connecteur comprenant une connexion électrique et/ou une connexion pneumatique additionnelle pour le conduit.

10. Un appareil (1) à utiliser dans un traitement de distribution de gaz humidifiés selon l'une quelconque des revendications précédentes, dans lequel ladite sortie côté patient inclut un connecteur pour recevoir un conduit respiratoire et soit une prise ou fiche de connexion électrique auxiliaire, soit une prise ou orifice de connexion pneumatique, pour réaliser une connexion simultanée lors de la connexion d'un circuit respiratoire ayant des connecteurs électriques ou pneumatiques complémentaires.

11. Un appareil (1) à utiliser dans un traitement de distribution de gaz humidifiés selon l'une quelconque des revendications précédentes, comprenant en outre le conduit respiratoire flexible, dans lequel le tube coudé (51) comprend une partie d'une terminaison du conduit respiratoire flexible.

12. L'appareil (1) à utiliser dans un traitement de distribution de gaz humidifiés selon la revendication 1, dans lequel la sortie de gaz sous pression (4) et le retour de gaz humidifiés (7) comprennent des tubes en saillie respectifs pour réaliser la connexion fluidique entre l'appareil et la chambre d'eau (2), dans lequel les tubes en saillie (4, 7) comprennent en outre une gaine élastique afin de fournir une étanchéité améliorée entre la chambre d'eau (2) et les parties en saillie (4, 7).

13. Un appareil (1) à utiliser dans un traitement de distribution de gaz humidifiés selon l'une quelconque des revendications précédentes, dans lequel l'alimentation en gaz sous pression comprend un ventilateur.

14. Un appareil (1) à utiliser dans un traitement de distribution de gaz humidifiés selon l'une quelconque des revendications précédentes, dans lequel ladite chambre d'eau (2) a une base, et ladite chambre d'eau (2) peut être mise en prise avec ledit boîtier par un mouvement unique.

15. Un appareil à utiliser dans un traitement de distribution de gaz humidifiés selon la revendication 14, où ledit mouvement unique de mise en prise pousse la base de ladite chambre d'eau (2) adjacente et en contact avec ledit moyen de chauffage de la chambre et réalise une première connexion fluidique entre ladite sortie de gaz sous pression et ladite entrée de la chambre d'eau, et réalise une deuxième connexion fluidique entre ledit retour de gaz humidifiés et ladite sortie (6) de la chambre d'eau, lesdites première et deuxième connexions fluidiques étant réalisées dans la direction dudit mouvement unique.

16. L'appareil (1) à utiliser dans un traitement de distribution de gaz humidifiés selon l'une quelconque des revendications précédentes, comprenant :
une chambre d'eau (2) ; et
un circuit respiratoire ayant des connecteurs électriques complémentaires.

17. L'appareil (1) selon l'une quelconque des revendications précédentes configuré pour la distribution de gaz humidifiés.

18. L'appareil (1) selon l'une quelconque des revendications précédentes configuré pour la thérapie CPAP.
